# EUROPEAN PATENT SPECIFICATION

(11) **EP 0 487 973 B1**
(45) Date of publication and mention of the grant of the patent: **27.03.1996**
(21) Application number: 91119316.7
(22) Date of filing: 13.11.1991
(51) Int. Cl.: A61K 31/60, A61K 47/10

(54) **Topical lotion containing Niclosamide**
Topisch anzuwendende Niclosamid enthaltende Lotion
Lotion à utilisation topique à base de Niclosamide

(30) Priority: 26.11.1990 US 617778; 17.09.1991 US 760819
(43) Date of publication of application: 03.06.1992
(73) Proprietor: Bayer Corporation, Pittsburgh, PA 15219-2502 (US)
(72) Inventor: Day, John I.E., Westport, CT 06880 (US); Ramineni, Rao E., Orange, CT 06477 (US)
(74) Representative: Dänner, Klaus

(56) References cited:
- GB-A- 2 213 722
- US-A- 4 659 738
- The Merck Index, 11th ed. (1989), page 1204

## Description

### Background of the Invention

The invention relates to a topical antipenetrant formulation for niclosamide which prevents the penetration of cercariae of Schistosoma into skin.

Schistosomiasis is a debilitating parasitic condition which affects a large number of people in the third world and is listed by the World Health Organization as the second worse health problem in the world. During World War II, whole units of men were seriously infected. The US Army has therefore been working to obtain an effective formulation to protect troops from infection by cercariae, the only stage in the parasite's life cycle capable of getting through the body's defenses.

Niclosamide is claimed in US Patent 3,079,297 for use against gastropods. It is currently marketed as YOMESAN® in a 500 mg chewable tablet by Bayer AG and as NICLOCIDE® 500 mg chewable tablet by Miles Inc.

In GB-A-2213722 anthelmintic niclosamide compositions are described and claimed in form of suspensions. The PEG 6000 is particularly mentioned as encapsulation agent for niclosamide in form of a dry powder, as shown in example 10 or in the form of a dispersion in water as shown in example 9.

US Patent No. 4,659,738, assigned to the US Army, disclosed that a solution containing from about 0.10% to about 3.0% weight of niclosamide to volume of vehicle provided effective topical protection from penetration by the parasite. Vehicles listed included solutions (as in alcoholic solution i.e. methanol, ethanol or isopropanol); dimethylsulfoxide; creams (as vanishing cream); ointment (as, white or yellow ointment); liniment (as, green soap tincture); or malagma (as an emollient oil). In the examples, the compound (or its analogues) was solubilized in absolute methanol, ethanol, dimethylsulfoxide or isopropanol. The Army has also published an article in Science, Vol. 246, 1242-1243, 8 December 1989, indicating their interest in the lotion of this invention; however, the formulation claimed herein was not disclosed.

To date, no topical formulation is available. Although niclosamide is physically stable, the drug is very water insoluble and therefore difficult to formulate into an acceptable lotion.

### Summary of the Invention

The invention provides a topical antipenetrant formulation for the prevention of infection by Schistosoma, comprising, by weight: from about 0.1 to about two percent niclosamide, and a polyethylene glycol chosen from the group consisting of polyethylene glycol 200, polyethylene glycol 400, polyethylene glycol 800 and polyethylene glycol 1000.

A preferred formulation is composed of about 1 percent niclosamide, about 40 to 55% percent propylene glycol, about 20 to 30% alcohol and about 20 to 25% polyethylene glycol 400; and from about 0.1 to 10% water.

### Description of the Invention

Niclosamide, 2',5-dichloro-4'-nitrosalicylanide, is a known agent which can provide topical antipenetrant protection against Schistosomiasis. Since niclosamide is very insoluble in water, crystallization or precipitation is a major formulation problem. Although niclosamide is soluble in ethanol, pure alcoholic solutions should not be applied directly to the skin over a prolonged period due to the drying effects. However, the addition of any appreciable water to an alcoholic solution of niclosamide in order to decrease the drying problem, causes precipitation which is not only undesirable esthetically in a topical formulation but also decreases the effectiveness. Therefore it is an object of the invention to provide a topical lotion formulation which effectively prevents the penetration of larvae of Schistosomes into skin and may be applied once or twice a day over a prolonged period without deleterious effects to the skin.

The present formulation fulfills that objective. Its usefulness has been tested with lower vertebrates and it is currently being tested in human clinical trials.

A useful lotion may be prepared with simply about 0.1 to 2 % niclosamide and a polyethylene glycol chosen from the group consisting of polyethylene glycol 200, 400, 800 or 1000. However although niclosamide is soluble in polyethylene glycol and such a formulation will provide a topical lotion which may be applied to the skin repeatedly over a prolonged period, vehicles of this type are greasy and slippery and therefore, by themselves, are undesirable esthetically in a topical formulation. Preferred formulations include propylene glycol or glycerin, an alcohol such as ethanol or isopropanol and water; in addition to polyethylene glycol. The most preferred formulation contains about 1% niclosamide; about 40 to 55 percent propylene glycol; about 20 to 30 percent ethanol; about 20 to 25 percent polyethylene glycol 400; and about 0.1 to 10% water. The addition of some water prevents the dehydration of the skin with prolonged use.

Other components including viscosity aids such as Carbopol and tetra (2-hydroxypropylethylenediamine) may also be added and are well known to those of skill in the art.

In use, the formulation is applied to areas of the body which may come in contact with water containing the Schistosome larvae, cercariae. This may be principally the feet and legs or may be a more extensive portion of the body if prolonged immersion in infected waters is expected. The lotion may be applied once or twice a day and will provide protection for up to six days even if the area is immersed in water.

The following example discloses preferred embodiment of the invention, but does not limit the applicability of the invention which is solely defined by the claims.

### EXAMPLE

| | % W/W |
|---|---|
| Niclosamide | 1.003 |
| Propylene Glycol | 48.043 |
| Ethanol, 95% | 25.476 |
| Polyethylene Glycol 400 | 23.972 |
| Carbopol 941® | 0.502 |
| Tetra (2-hydroxypropyl) Ethylene Diamine | 0.502 |
| Purified Water | 0.502 |

Mix ethanol, propylene glycol, carbopol and tetra (2-hydroxypropyl) ethylene diamine and water. Dissolve Niclosamide in polyethylene glycol and mix with the above alcoholic solution.

It should be understood that many modifications and variations can be made in the proportions and components used herein without departing from the scope of the invention, which is solely defined by the claims.

## Claims (Claims for the following Contracting State(s): AT, BE, CH, DE, DK, FR, GB, IT, LI, LU, NL, SE)

1. A topical antipenetrant lotion formulation for the prevention of infection by Schistosoma, composed of between 0.1 and 2 % niclosamide and a polyethylene glycol chosen from the group consisting of polyethylene glycol 200, 400, 800 and 1000.

2. The topical antipenetrant formulation of claim 1, which additionally contains propylene glycol, an alcohol and water.

3. A topical antipenetrant formulation of niclosamide, composed of
a. between about 0.1 and about 2% niclosamide;
b. between about 20 to 25% of a polyethylene glycol chosen from the group consisting of polyethylene glycol 200, 400, 800 and 1000;
c. between about 40 to 55% propylene glycol or glycerin;
d. between about 20 to 30% ethanol or isopropanol; and
e. between about 0.1 and about 10% water.

4. The topical antipenetrant formulation of claim 3, composed of
a. 1% niclosamide;
b. 24% polyethylene glycol 400;
c. 49% propylene glycol;
d. 25% ethanol; and
e. 1% water.

## Claims (Claims for the following Contracting State(s): ES, GR)

1. Process for manufacturing of a topical antipenetrant lotion formulation for the prevention of infection by Schistosoma, composed of between 0.1 and 2 % niclosamide and a polyethylene glycol chosen from the group consisting of polyethylene glycol 200, 400, 800 and 1000, characterized that niclosamide is dissolved in polyethylene glycol and optionally mixed with additional alcohols and water.

## Patentansprüche (Patentansprüche für folgende(n) Vertragsstaat(en): AT, BE, CH, DE, DK, FR, GB, IT, LI, LU, NL, SE)

1. Topische nicht-eindringende Lotions-Formulierung zur Verhinderung von Bilharzia-Infektionen, zusammengesetzt aus zwischen 0,1 und 2 % Niclosamid und einem Polyethylenglykol, ausgewählt aus der Gruppe bestehend aus Polyethylenglykol 200, 400, 800 und 1000.

2. Topische nicht-eindringende Formulierung gemäß Anspruch 1, die zusätzlich Propylenglykol, einen Alkohol und Wasser enthält.

3. Topische nicht-eindringende Formulierung von Niclosamid, zusammengesetzt aus:
a. zwischen etwa 0,1 und etwa 2 % Niclosamid;
b. zwischen etwa 20 und 25 % eines Polyethylenglykols ausgewählt aus der Gruppe bestehend aus Polyethylenglykol 200, 400, 800 und 1000;
c. zwischen etwa 40 und 55 % Propylenglykol oder Glycerin;
d. zwischen etwa 20 und 30 % Ethanol oder Isopropanol; und
e. zwischen etwa 0,1 und 10 % Wasser.

4. Topische nicht-eindringende Formulierung gemäß Anspruch 3, zusammengesetzt aus:
a. 1 % Niclosamid;
b. 24 % Polyethylenglykol 400;
c. 49 % Propylenglykol;
d. 25 % Ethanol; und
e. 1 % Wasser.

## Patentansprüche (Patentansprüche für folgende(n) Vertragsstaat(en): ES, GR)

1. Verfahren zur Herstellung einer topischen nichteindringenden Lotions-Formulierung zur Verhinderung von Schistosoma-Infektionen, zusammengesetzt aus zwischen 0,1 und 2 % Niclosamid und einem Polyethylenglykol, ausgewählt aus der Gruppe bestehend aus Polyethylenglykol 200, 400, 800 und 1000, dadurch **gekennzeichnet,** dass Niclosamid in Polyethylenglykol gelöst und wahlweise mit zusätzlichen Alkoholen und Wasser vermischt wird.

## Revendications (Revendications pour l'(les) Etat(s) contractant(s) suivant(s): AT, BE, CH, DE, DK, FR, GB, IT, LI, LU, NL, SE)

1. Formulation de lotion antipénétrante topique pour la prévention d'une infection par Schistosoma, constituée de 0,1 à 2 % de niclosamide et d'un polyéthylèneglycol choisi dans le groupe consistant en les polyéthylèneglycols 200, 400, 800 et 1000.

2. Formulation antipenétrante topique suivant la revendication 1, qui contient en outre du propylèneglycol, un alcool et de l'eau.

3. Formulation antipénétrante topique de niclosamide, constituée
a. d'environ 0,1 à environ 2 % de niclosamide ;
b. d'environ 20 à 25 % d'un polyéthylèneglycol choisi dans le groupe consistant en les polyéthylèneglycols 200, 400, 800 et 1000 ;
c. d'environ 40 à 55 % de propylèneglycol ou de glycérol ;
d. d'environ 20 à 30 % d'éthanol ou d'isopropanol ; et
e. d'environ 0,1 à environ 10 % d'eau.

4. Formulation antipénétrante topique suivant la revendication 3, constituée
a. de 1 % de niclosamide ;
b. de 24 % de polyéthylèneglycol 400 ;
c. de 49 % de propylèneglycol ;
d. de 25 % d'éthanol ; et
e. de 1 % d'eau.

## Revendications (Revendications pour l'(les) Etat(s) contractant(s) suivant(s): ES, GR)

1. Procédé de production d'une formulation de lotion antipénétrante topique pour la prévention d'une infection par Schistosoma, constituée de 0,1 à 2 % de niclosamide et d'un polyéthylèneglycol choisi dans le groupe consistant en les polyéthylèneglycols 200, 400, 800 et 1000, caractérisé en ce que le niclosamide est dissous dans le polyéthylèneglycol et mélangé facultativement à des alcools supplémentaires et de l'eau.
